# EUROPEAN PATENT SPECIFICATION

(11) **EP 2 887 070 B1**
(45) Date of publication and mention of the grant of the patent: **18.04.2018**
(21) Application number: 14198959.0
(22) Date of filing: 18.12.2014
(51) Int. Cl.: G01N 33/94, C07K 16/44

(54) **Tilidine immunodetection**
Tilidin-Immundetektion
Immunodetection de Tilidine

(30) Priority: 23.12.2013 GB 201322935
(43) Date of publication of application: 24.06.2015
(73) Proprietor: Randox Laboratories Ltd., Crumlin, County Antrim BT29 4QY (GB)
(72) Inventor: Benchikh, Elouard, Crumlin, Antrim BT29 4QY (GB); Fitzgerald, Peter, Crumlin, Antrim BT29 4QY (GB); Lowry, Philip, Crumlin, Antrim BT29 4QY (GB); McConnell, Ivan, Crumlin, Antrim BT29 4QY (GB)

(56) References cited:
- EP-A1- 1 988 398
- CHRISTOPH KÖHLER ET AL: "Rapid quantification of tilidine, nortilidine, and bisnortilidine in urine by automated online SPE-LC-MS/MS", ANALYTICAL AND BIOANALYTICAL CHEMISTRY, vol. 400, no. 1, 8 December 2010 (2010-12-08), pages 17-23, XP055180193, ISSN: 1618-2642, DOI: 10.1007/s00216-010-4466-1
- JOHANNA WEISS ET AL: "In vitro metabolism of the opioid tilidine and interaction of tilidine and nortilidine with CYP3A4, CYP2C19, and CYP2D6", NAUNYN-SCHMIEDEBERG'S ARCHIVES OF PHARMACOLOGY, SPRINGER, BERLIN, DE, vol. 378, no. 3, 31 May 2008 (2008-05-31), pages 275-282, XP019620946, ISSN: 1432-1912
- H Hengy ET AL: "Gas-chromatographic determination of nanogram amounts of enantiomers of nortilidine, a main metabolite of tilidine, in biological specimens", Clinical chemistry, 1 April 1978 (1978-04-01), pages 692-697, XP055180195, UNITED STATES Retrieved from the Internet: URL:http://www.ncbi.nlm.nih.gov/pubmed/639 277 [retrieved on 2015-03-30]

## Description

### Background

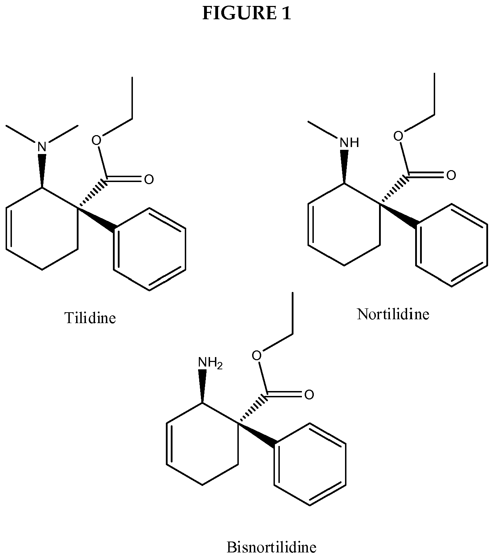

Tilidine, systematic name (±)-*trans*-ethyl 2-(N,N-dimethylamino)-1-phenylcyclohex-3-enylcarboxylate, is a synthetic opioid analgesic, commonly prescribed for acute and chronic pain. As the systematic name implies, it is a racemic mixture of the *1S*,*2R* and *1R*,*2S* compounds, the two trans-configured stereoisomers. Upon ingestion, it is rapidly metabolised undergoing demethylation to the pharmacologically active nortilidine, systematic name (±)-*trans*-ethyl 2-(N-methylamino)-1-phenylcyclohex-3-enylcarboxylate; the minor metabolite (±)-*trans*-ethyl 2-amino-1-phenylcyclohex-3-enylcarboxylate, trivial name bisnortilidine is also formed (Figure 1). Its opioid activity has led to its abuse and there is a need for its detection for toxicological purposes e.g. in individuals who drive while intoxicated, and for therapeutic drug monitoring. Various methods of detecting tilidine and its metabolites, mostly based on expensive, relatively complex laboratory-based mass-spectrometry and chromatographic techniques, are described e.g. Kohler et al (Anal.Bioanal. Chem., 2011, 400:17-23) describe a liquid chromatography-mass-spectrometry (LC-MS-MS) system. However, more sensitive, cheaper and simpler detection methods amenable to application in the field are desirable.

### Figures

Figure 1 Structure of tilidine, nortilidine and bisnortilidine
Figure 2 Preparation of hapten N-(carboxypropyl)nortilidine

### Description of the Invention

The invention describes a method of detecting or determining tilidine and nortilidine in an *in vitro* sample of an individual comprising; contacting the sample with one or more detecting agents and one or more antibodies; detecting, or determining the quantity of the one or more detecting agents; and deducing from calibrators the presence of or amount of tilidine and nortilidine in the sample or solution, the one or more antibodies characterised by having been derived from an immunogen of formula I 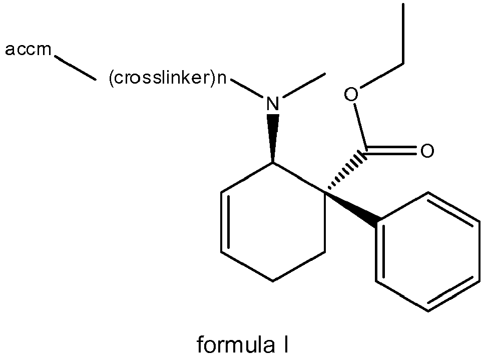 wherein n=0 or 1; when n=1 the crosslinker joins the nitrogen atom to the accm, the antigenicity conferring carrier material, and the crosslinker is -X-Y- in which X which is attached to the nitrogen is selected from a group comprising a substituted or unsubstituted, straight or branched chain, saturated or unsaturated C₁-C₆ alkylene moiety, and Y is selected from -C(O)-, -NH-, maleimide, -N-C(O)-, -N-C(S)-, -S-, -S(O)₂-, and - S(O)-. By 'detecting' is meant qualitatively analysing for the presence or absence of a substance. By 'determining' is meant quantitatively analysing for the amount of substance present. For convenience, throughout this specification, tilidine is represented as the *1S*,*2R* trans isomer; whenever the *1S*,*2R* trans isomer is depicted the *1R*,*2S* is also embraced unless otherwise stated. Hence formula I implies a mixture of the *1S*,*2R* and *1R,2S trans* isomers; although a racemate, i.e. 1:1 ratio of enantiomers, is the preferred mixture of the invention, variations which deviate from the 1:1 ratio of each *trans* enantiomer are also readily applied to the invention. It would be recognised by the skilled person that preparative isolation of either of the *trans* isomers of tilidine or a derivative of tilidine, or by using asymmetric synthetic methods, or both, enantiomeric immunogens could be produced for raising antibodies which would bind to tilidine and its metabolites. As the antibodies of the invention are able to bind tilidine and nortilidine, quantitative analysis will take the form of measuring the calibrator-equivalent amount. Any suitable *in vitro* biological sample may be used, but blood and urine are preferred.

The accm, is a standard component of an immunogen derived from a small molecule and can be any macromolecule that confers immunogenicity to the non-immunogenic small molecule (see Methods and Results section) but is preferably chosen from keyhole limpet haemocyanin (KLH), bovine thyroglobulin (BTG), bovine serum albumin (BSA), egg ovalbumin, bovine gamma globulin or cationised BSA.
The invention further describes an antibody derived from an immunogen of formula I. The antibody is further characterised in being specific to an epitope of tilidine. By specific it is meant that the antibody preferably binds or has greater affinity for tilidine compared to all other molecules. The relative degree of binding of an antibody to a molecule can be described by its cross-reactivity (CR) which can be derived from a suitable metric such as the IC₅₀ (see Table 1), the molecule with the greatest amount of binding being given a CR value of 100%. Thus the antibody of the current invention has a CR of 100% to tilidine. The antibody preferably is cross-reactive to nortilidine. The extent of cross-reactivity of the antibody to nortilidine is preferably >10 % more preferably >20%. Preferably, the antibody also shows cross-reactivity to bisnortilidine.

In a further embodiment the disclosure describes a kit for detecting or determining tilidine and nortilidine; the kit comprises at least one antibody specific to tilidine and having cross-reactivity to nortilidine. The kit may also contain a detecting agent comprising a hapten based on the tilidine structure. The detecting agent comprises a suitable hapten, preferably the haptens disclosed herein, covalently bonded to a detectable labelling agent, the hapten moiety being able to bind to the antibodies of the invention. Preferably, the labelling agent is selected from an enzyme, a luminescent substance, a radioactive substance, or a mixture thereof. More preferably, the labelling agent is an enzyme, preferably a peroxidase, most preferably horseradish peroxidise (HRP). The kit may comprise one or more antibodies of the invention and one or more additional antibodies with different molecular specificities i.e. these additional antibodies do not bind to the same structural epitopes as the antibodies of the invention. Such an arrangement enables a multiplexing approach to the detection or determination of drugs of abuse. The multiplexing approach preferably makes use of a planar substrate to which the antibodies are attached, such as a ceramic chip or an appropriately surface-modified glass slide. Beads may also be used.

### Methods and Results

### Preparation of Haptens, Immunogens and Detecting Agents

The general process of immunogen formation is well known. Briefly, a hapten (a pre-immunogenic structure) whose structure incorporates the structural epitope(s) to be recognized by the antibody, is synthesized by introducing a crosslinker. The crosslinker provides the functional groups which will enable an antigenicity conferring carrier material (accm) to be attached to the hapten. The crosslinker is not always necessary if it is considered that the accm can be directly attached to an existing molecule which incorporates the required structural elements for immunogen attachment. The crosslinker may also be transient in nature, being a structural group which activates the hapten to accm introduction but which is not present in the final immunogen (e.g. N,N-dicyclohexylcarbodiimide and N-hydroxysuccinimide). Further examples of crosslinking agents include 1-cyclohexyl-3-(2-morpholino-yl-ethyl)carbodiimide, 1-ethyl-3-(3-dimethylaminopropyl)carbodiimide (EDC), 2-iminothiolane, 3-maleimidobenzoic acid , glutardialdehyde, succinimidyl 4-(N-maleimidomethyl)cyclohexane-1-carboxylate, N-succinimidyl-3-(2-pyridyldithio) propionate,. A comprehensive list of crosslinkers can be found in the Thermo Scientific Pierce Crosslinking Technical Handbook, 2009. As previously described, although small molecules (haptens) provide defined structural epitopes, they are not in themselves immunogenic and therefore need to be conjugated to carrier materials, which will elicit an immunogenic response when administered to a host animal.

Appropriate carrier materials commonly contain poly(amino acid) segments and include polypeptides, proteins and protein fragments. Illustrative examples of useful carrier materials are BSA, egg ovalbumin, bovine gamma globulin, cationized serum albumin, BTG, KLH, dextranes etc. Alternatively, synthetic poly(amino acids) having a sufficient number of available amino groups, such as lysine, may be employed, as may other synthetic or natural polymeric materials bearing reactive functional groups. Also, carbohydrates, yeasts or polysaccharides may be conjugated to the hapten to produce an immunogen (Bioconjugate Techniques, G. Hermanson, ed, Academic Press, 1996, 785pp, lists common carrier proteins). The haptens can also be coupled to a detectable labelling agent such as an enzyme (for example, horseradish peroxidase), a substance having fluorescent properties or a radioactive label for the preparation of detecting agents for use in the immunoassays. The fluorescent substance may be, for example, a monovalent residue of fluorescein or a derivative thereof. Immunogen formation for the invention described herein involves conventional conjugation chemistry. In order to confirm that adequate conjugation of hapten to carrier material has been achieved, prior to immunisation, each immunogen is evaluated using matrix-assisted UV laser desorption /ionisation time-of-flight mass spectroscopy (MALDI-TOF MS). Formation of the conjugate (detecting agent) from a hapten follows similar principles to immunogen formation.

### General Procedure for MALDI-TOF Analysis of Immunogens.

MALDI-TOF mass spectrometry was performed using a Voyager STR Biospectrometry Research Station laser-desorption mass spectrometer coupled with delayed extraction. An aliquot of each sample to be analysed was diluted in 0.1 % aqueous trifluoroacetic acid (TFA) to create 1mg/ml sample solutions. Aliquots (1µl) were analysed using a matrix of sinapinic acid and bovine serum albumin (Fluka) was used as an external calibrant.

### Preparation of Antisera

In order to generate polyclonal antisera, an immunogen of the present invention is mixed with Freund's adjuvant and the mixture is injected into a host animal, such as rabbit, sheep, mouse, guinea pig or horse. Sheep are the preferred host animal. Further injections (boosts) are made and serum is sampled for evaluation of the antibody titre. When the optimal titre has been attained, the host animal is bled to yield a suitable volume of specific antiserum. The degree of antibody purification required depends on the intended application. For many purposes, there is no requirement for purification, however, in other cases, such as where the antibody is to be immobilised on a solid support, purification steps can be taken to remove undesired material and eliminate non-specific binding.

### Immunoassay Development

The process of developing an immunoassay is well known to the person skilled in the art. Briefly, for a competitive immunoassay in which the target analyte is a non-immunogenic molecule such as a hapten, the following process is conducted: antibodies are produced by immunising an animal, preferably a mammalian animal, by repeated administration of an immunogen. The serum from the immunised animal is collected when the antibody titre is sufficiently high. A detecting agent is added to a sample containing the target analyte and the raised antibodies, and the detecting agent and analyte compete for binding to the antibodies. The process may comprise fixing said serum antibodies to a backing substrate such as a microtitre plate, a polystyrene solid support or a ceramic chip. The antibodies can be polyclonal or monoclonal. The signal emitted in the immunoassay is proportionate to the amount of detecting agent bound to the antibodies which in turn is inversely proportionate to the analyte concentration. The signal can be detected or quantified by comparison with a calibrator.

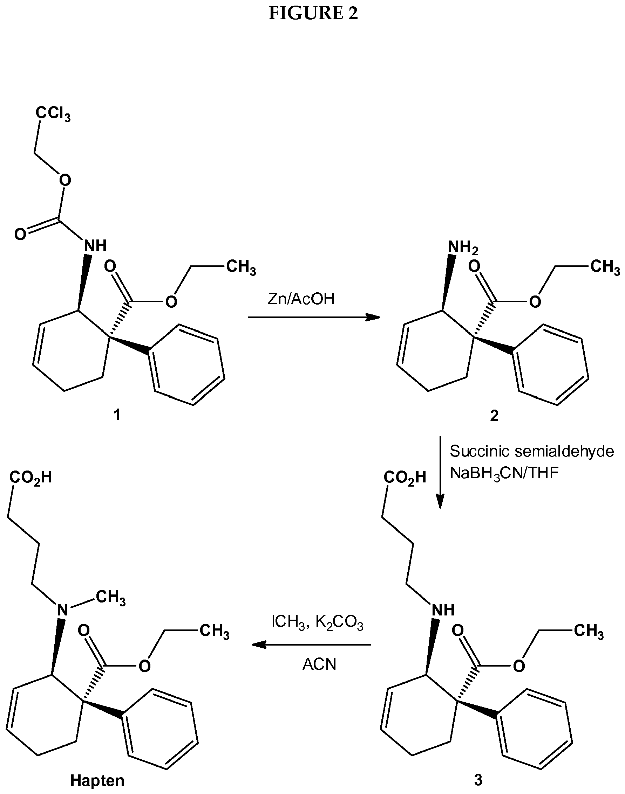

In the following Examples, the numbers following chemical names refer to the structure of Figure 2

### Example 1: Preparation of bisnortilidine 2.

Zinc activation: Zn (5g) was washed successively with HCl (2%), water, ethanol and finally with ether before being dried under high vacuum. To a solution of N-(2,2,2-trichloroethoxy)carbonyl bisnortilidine **1** (900mg, 2.14mmol; Toronto Research Chemicals Inc.) in acetic acid (10.8ml) at room temperature was added Zinc dust activated (1.43g, 0.021mol) portion-wise and the resulting mixture was stirred at room temperature overnight. The reaction mixture was filtered and washed by acetic acid. The combined acetic acid was removed in vacuo and the resulting residue was taken into NaOH (2N) solution and extracted several times with chloroform. The organic layer were dried over sodium sulfate, filtered and concentrated to dryness to give 593.5mg of bisnortilidine **2** as clear oil.

### Example 2: Preparation of N-(carboxypropyl)bisnortilidine 3

To a cooled solution 0°C of bisnortilidine **2** (469mg, 1.91mmol) in anhydrous tetrahydrofuran (3ml) was added aqueous succinic semialdehyde (1.69ml, 2.48mmol) followed by the addition of sodium cyanoborohydride (120mg, 1.19mmol). The reaction mixture was stirred for 2 hours. To this solution was added a solution of HCl (1N) (3ml) and the reaction mixture stirred at room temperature for 2 hours. The reaction mixture was concentrated in vacuo and the residue made neutral with a solution of NaOH (1N) and extracted with chloroform. The aqueous phase was concentrated in vacuo and extracted with (10%) methanol in chloroform. The organic fractions were combined and evaporated to give 331.7mg of N-(carboxypropyl)bisnortilidine **3.**

### Example 3: Preparation of N-(carboxypropyl)nortilidine (Hapten)

To solution of N-(carboxypropyl)bisnortilidine **3** (331.7mg, 1mmol) in acetonitrile (ACN) (2ml) was added potassium carbonate (317mg, 2.3mmol) and methyl iodide (93.4µl, 1.5mmol) and the reaction mixture was stirred at room temperature overnight. The solution was concentrated in vacuo and the residue was taken in water. The mixture was neutralized the pH7 with HCl (1N) and extracted with ethyl acetate. The combined organic layers were dried over sodium sulfate, filtered and concentrated to dryness. The residue was purified by column chromatography (silica gel, 10% methanol in chloroform) to give 146mg of N-(carboxypropyl)nortilidine (Hapten) as oil.

### Example 4: Conjugation of N-(carboxypropyl)nortilidine (Hapten) to BSA (Immunogen-I)

To a solution of N-(carboxypropyl)nortilidine (Hapten) (38.7mg, 0.112mmol) in DMF (1.0ml) was added N,N-dicyclohexylcarbodiimide (DCC) (25.35mg, 0.123mmol) and N-hydroxysuccinimide (14.13mg, 0.123mmol) and the mixture stirred at room temperature overnight. The dicyclohexylurea formed was removed by filtration and the solution was added dropwise to a solution of BSA (150mg, 2.3mmol) in 50mM sodium bicarbonate solution (pH 8.5) (10ml). The mixture was then stirred overnight at 4°C. The solution was then dialysed against 50mM phosphate buffer pH 7.2 (3 changes) for 24 hours at 4°C, and freeze-dried. MALDI results showed 24.99 molecule of N-(carboxypropyl)nortilidine (Hapten) had been conjugated to one molecule of BSA.

### Example 5: Conjugation of N-(carboxypropyl)nortilidine (Hapten) to BTG

**(Immunogen-II)** To a solution of N-(carboxypropyl)nortilidine (Hapten) (46.63mg, 0.135mmol) in DMF (1.0ml) was added N,N-dicyclohexylcarbodiimide (DCC) (30.7mg, 0.149mmol) and N-hydroxysuccinimide (17.13mg, 0.149mmol) and the mixture stirred at room temperature overnight. The dicyclohexylurea formed was removed by filtration and the solution was added dropwise to a solution of BTG (150mg, 2.25umol) in 50mM sodium bicarbonate solution (pH 8.5) (10ml). The mixture was then stirred overnight at 4°C. The solution was then dialysed against 50mM phosphate buffer pH 7.2 (3 changes) for 24 hours at 4°C, and freeze-dried.

### Example 6: Conjugation of N-(carboxypropyl)tilidine (Hapten) to HRP

EDC hydrochloride (10mg) was dissolved in water (0.5ml) and immediately added to a solution of N-(carboxypropyl)tilidine (Hapten) (2mg) in DMF (0.2ml). After mixing, this solution was added dropwise to a solution of HRP (20mg) in water (1ml). Sulfo-NHS (5mg) was added and the reaction mixture was incubated in the dark at room temperature overnight. Excess hapten was removed with double PD-10 columns (Pharmacia) in series, pre-equilibrated with PBS at pH 7.2. The hapten-HRP conjugate was then dialysed overnight against 10L of PBS at pH 7.2 at 4°C.

### Example 7: Immunoassay development

The wells of an enhanced binding 96 well polystyrene microtitre plate were coated with the Ig fraction of the antiserum raised to Immunogen II (tilidine-BTG), diluted in 10mMTris, pH 8.5 (125µl/well). The appropriate antibody coating dilution was determined using standard ELISA checkerboard techniques. The plate was incubated for 2 hours at 37°C, washed 4 times over 10 minutes with Tris buffered saline, containing Tween 20 (TBST) and tapped dry. Standard solutions of tilidine hydrochloride and nortilidine hydrochloride were prepared in TBST at 0, 0.625ng/ml, 1.25ng/ml, 2.5ng/ml, 5ng/ml, 10ng/ml, 20ng/ml and 40ng/ml and 50µl of each was added to the appropriate wells (Figure 1). 75µl of conjugate of example 6 (hapten-HRP), diluted in Tris buffer (pH7.2) containing EDTA, D-mannitol, sucrose, thimerosal and BSA was added to each of the wells. The appropriate dilution of conjugate was also determined using standard ELISA checkerboard techniques. The plate was incubated at 25°C for 1 hour. Excess unbound conjugate was removed by washing 6 times over a 10-15 minute period with TBST. 125µl of tetramethylbenzidine (TMB) substrate solution was added to each well of the plate that was then incubated for 20 minutes in the dark at room temperature. The reaction was terminated by the addition of 125µl of 0.2M H₂SO₄ to each well. The absorbance was then measured at 450nm using a microtitre plate reader.

**Table 1 Binding characteristics of tilidine-specific antibody raised from Immunogen -II using conjugate of Example 6 as detecting agent**

| | Tilidine | | Nortilidine | |
|---|---|---|---|---|
| Standard ng/ml | A450 | % B/Bo | A450 | % B/Bo |
| 0 | 1.561 | 100 | 1.551 | 100 |
| 0.625 | 0.989 | 63 | 1.227 | 79 |
| 1.250 | 0.861 | 55 | 1.129 | 73 |
| 2.500 | 0.714 | 46 | 1.004 | 65 |
| 5.000 | 0.572 | 37 | 0.904 | 58 |
| 10.000 | 0.444 | 28 | 0.745 | 48 |
| 20.000 | 0.319 | 20 | 0.571 | 37 |
| 40.000 | 0.212 | 14 | 0.426 | 27 |
| IC₅₀ ng/ml | 1.820 | | 8.522 | |
| % cross-reactivity | 100 | | 21.36 | |

| | | | | |
|---|---|---|---|---|
| A₄₅₀ = absorbance at 450nm; B= absorbance at 450nm at x ng/ml standard concentration; Bo= absorbance at 450nm at 0 ng/ml standard concentration; IC₅₀= standard concentration which produces 50% B/B | | | | |

Repetition of Example 7, in which the nortilidine standard was replaced by bisnortilidine hydrochloride, showed bisnortilidine to have a cross-reactivity of 15.40 % compared to tilidine (100 %).

## Claims

1. A competitive immunoassay method of detecting or determining tilidine and nortilidine in an *in vitro* sample of an individual comprising; contacting the sample with one or more detecting agents and one or more antibodies, the detecting agent(s) competing with tilidine and nortilidine for binding to the antibodies; detecting or determining the quantity of the one or more detecting agents; and deducing from calibrators the presence of or amount of tilidine and nortilidine in the sample or solution, the one or more antibodies **characterised by** having been derived from an immunogen of formula I 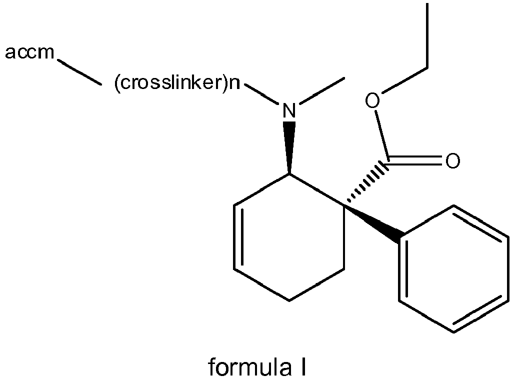 wherein n=0 or 1; when n=1 the crosslinker joins the nitrogen atom to the accm, the antigenicity conferring carrier material, and the crosslinker is -X-Y- in which X which is attached to the nitrogen atom is selected from a substituted or unsubstituted, straight or branched chain, saturated or unsaturated C₁-C₆ alkylene moiety, and Y is selected from -C(O)-, -NH-, maleimide, -N-C(O)-, -N-C(S)-, -S-, -S(O)₂-, and -S(O)-.

2. An antibody specific to an epitope of tilidine further **characterised by** being derived from an immunogen of formula I of claim 1.

3. The antibody of claim 2 which has a cross-reactivity of >10% to nortilidine.

4. A kit for detecting or determining tilidine and nortilidine, the kit comprising at least one antibody of either of claims 2 or 3.

## Patentansprüche

1. Ein kompetitives Immunoassay-Verfahren zum Nachweis oder zur Bestimmung von Tilidin und Nortilidin in einer In-vitro-Probe eines Individuums, umfassend; Inkontaktbringen der Probe mit einem oder mehreren Nachweismitteln und einem oder mehreren Antikörpern, wobei das Nachweismittel mit Tilidin und Nortilidin um die Bindung an die Antikörper konkurriert; Detektieren oder Bestimmen der Menge des einen oder der mehreren Detektionsmittel; und Ableiten des Vorhandenseins oder der Menge von Tilidin und Nortilidin in der Probe oder Lösung aus Kalibratoren, wobei der eine oder die mehreren Antikörper **dadurch gekennzeichnet sind, dass** sie von einem Immunogen der Formel I abgeleitet sind 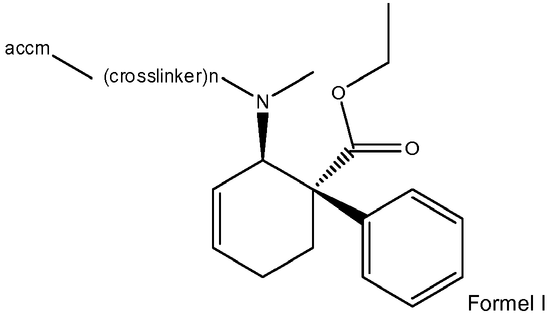 worin n = 0 oder 1 ist; wenn n = 1 ist, der Crosslinker das Stickstoffatom mit dem accm verbindet, dem die Antigenität verleihenden Trägermaterial, und der Crosslinker ist -XY-, worin X, das an das Stickstoffatom gebunden ist, ausgewählt ist aus einer substituierten oder unsubstituierten, geraden oder verzweigten Kette, gesättigte oder ungesättigte C₁-C₆-Alkyleneinheit, und Y ist ausgewählt aus -C(O)-, -NH-, Maleinimid, -N-C(O)-, -N-C(S)-, -S-, -S(O)₂- und -S(O)-.

2. Ein Antikörper, der für ein Epitop von Tilidin spezifisch ist, ferner **dadurch gekennzeichnet, dass** er von einem Immunogen der Formel I von Anspruch 1 abgeleitet ist.

3. Antikörper nach Anspruch 2, der eine Kreuzreaktivität von> 10% zu Nortilidin aufweist.

4. Kit zum Nachweis oder zur Bestimmung von Tilidin und Nortilidin, wobei der Kit mindestens einen Antikörper nach einem der Ansprüche 2 oder 3 enthält.

## Revendications

1. Méthode immunologique compétitive de détection ou de détermination de la tilidine et de la nortilidine dans un échantillon in vitro d'un individu comprenant; mettre en contact l'échantillon avec un ou plusieurs agents de détection et un ou plusieurs anticorps, le ou les agents de détection entrant en compétition avec la tilidine et la nortilidine pour se lier aux anticorps; détecter ou déterminer la quantité du ou des agents de détection; et déduire des étalons la présence ou la quantité de tilidine et de nortilidine dans l'échantillon ou la solution, le ou les anticorps **caractérisés en ce qu'**ils ont été dérivés d'un immunogène de formule I 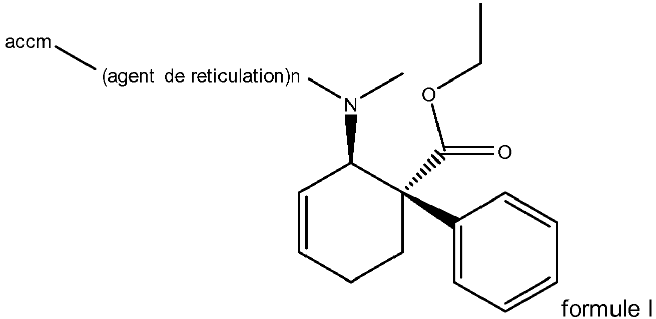 où n = 0 ou 1; lorsque n = 1, l'agent de réticulation relie l'atome d'azote à l'accm, le véhicule porteur conférant l'antigénicité, et l'agent de réticulation est -XY- dans lequel X qui est lié à l'atome d'azote est choisi parmi une chaîne linéaire ou ramifiée substituée ou non substituée, un fragment alkylène en C₁-C₆ saturé ou insaturé, et Y est choisi parmi -C(O)-, -NH-, maléimide, -N-C(O)-, -N-C(S)-, -S-, -S(O)₂-, et -S(O)-.

2. Anticorps spécifique d'un epitope de la tilidine **caractérisé en outre par le fait qu'**il est dérivé d'un immunogène de formule I selon la revendication 1.

3. Anticorps selon la revendication 2, qui a une réactivité croisée de> 10% à la nortilidine.

4. Kit de détection ou de détermination de la tilidine et de la nortilidine, le kit comprenant au moins un anticorps de l'une quelconque des revendications 2 ou 3.
